(19) Europäisches Patentamt
European Patent Office
Office européen des brevets

(11) Publication number: **0 047 122**
**A2**

(12) # EUROPEAN PATENT APPLICATION

(21) Application number: 81303870.0

(22) Date of filing: 25.08.81

(51) Int. Cl.$^3$: **A 61 K 9/00**
A 61 K 31/415, C 07 D 235/30

(30) Priority: 28.08.80 US 182299

(43) Date of publication of application: 10.03.82 Bulletin 82/10

(84) Designated Contracting States: BE CH DE FR GB IT LI LU NL SE

(71) Applicant: ELI LILLY AND COMPANY
307, East McCarty Street
Indianapolis Indiana 46206(US)

(72) Inventor: Su, Kenneth Shyan-Ell
8530 Trails Run Road
Indianapolis Indiana 46217(US)

(74) Representative: Crowther, Terence Roger et al,
Erl Wood Manor
Windlesham Surrey GU20 6PH(GB)

(54) **Intranasal formulation.**

(57) Intranasal formulations for antiviral benzimidazole compounds are disclosed. The formulations contain the active ingredient; a non-ionic, surface-active agent with a HLB number from 12 to 16; and a propellant.

EP 0 047 122 A2

Croydon Printing Company Ltd.

# INTRANASAL FORMULATION

A number of substituted benzimidazoles have been discovered that display unusually good antiviral activity, but are very insoluble; see for example U.S. Patents 4,008,243; 4,018,790; 4,118,573; and 4,118,742. Among the most active of such benzimidazole antiviral agents are a group of oximes, which are 1-substituted-sulfonyl-2-amino-5(6)-substituted-iminobenzimidazoles. These oximes are especially active against rhinoviruses and respiratory infections. Intranasal formulations are, therefore, desirable for these compounds, because such formulations minimize systemic absorption and concentrate the antiviral compound in a target area, the nose.

This invention provides an intranasal formulation for these insoluble benzimidazoles, which formulation possesses suitable bioavailability and shelf life. In addition, the formulation possesses suitable stability and causes minimal isomerization for those benzimidazole compounds which have isomers, like the oximes. Minimal isomerization is important, because for some of these compounds one isomer is more active than the other. For example, the anti isomer of 1-isopropylsulfonyl-2-amino-6-(α-hydroxyiminobenzyl)benzimidazole is about eight times more potent than the syn isomer.

Although intranasal and inhalation aerosol formulations are known in the art, none of the known formulations describe the present invention. See Derwent Abstracts: 66454A/37; 13119X/08; 06047B/04; U.S. Patents 3,014,844; and 4,213,993.

Specifically, this invention provides an intranasal formulation which comprises:

a) from about .01 to about 1.0% of a compound having the formula (I)

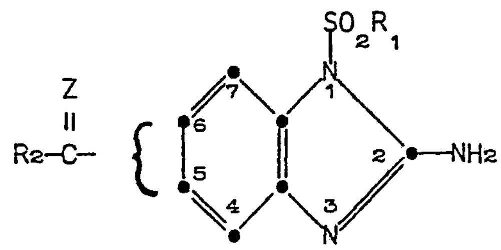

(I)

wherein $R_1$ is $C_1$-$C_5$ alkyl, $C_3$-$C_7$ cycloalkyl, or thienyl;

$R_2$ is hydrogen, $C_1$-$C_7$ alkyl, $C_3$-$C_7$ cycloalkyl, or phenyl;

Z is oxo, hydroxyimino, $C_1$-$C_4$ acyloxyimino, or $C_1$-$C_7$ alkylidene; and

$R_2-\overset{Z}{\overset{\|}{C}}-$ is at the 5 or 6 position;

such compound of formula (I) having a particle size from about 5 to about 200 microns;

b) from about 15.0 to about 50.0% of a pharmaceutically-acceptable, non-ionic, surface-active agent with a HLB number from about 12 to about 16;

c) from 0.0 to about 25.0% of a pharmaceutically-acceptable, solvent selected from ethanol, propylene glycol, and liquid polyethylene glycol; and

d) from about 40.0 to about 80.0% of a propellant.

The present invention relates to new pharmaceutical intranasal formulations, as defined above.

The preparation of the compounds of formula I used in these formulations is described in U.S. Patent 4,118,742, which is incorporated by reference. The separation of syn- and anti-oximes is described in U.S. Patent 4,191,832, which is also incorporated by reference.

The particle size of the compound should be from about 5 to about 200 microns, with the preferred particle size from about 5 to about 20 microns.

Particular surface-active agents or combinations of agents are required to give desirable results. The non-ionic, surface-active agent employed should have an hydrophile-lipophile balance (HLB) number of between about 12 and about 16. The HLB number is an empirical number, which provides a guide to the surface-active properties of a surface-active agent. The lower the HLB number, the more lipophilic is the agent, and conversely, the higher the HLB number, the more hydrophilic is the agent. The HLB number is well-known and understood and its method of determination is described by W.C. Griffin in the Journal of the Society of Cosmetic Chemists, Vol. 1, No. 5, pages 311-326 (1949). It is possible to employ surface-active agents which themselves do not possess an HLB number within these ranges, providing they are used in conjunction with other surface-active agents, the combination of which provides a mixture having an HLB number within the prescribed range.

Those surface-active agents that are soluble or dispersible in the propellant are effective with the most effective agents being the most propellant soluble. For medicinal use it is also important that the surface-active agent should be non-irritating and non-toxic.

Among the non-ionic surface-active agents which may be employed are: non-ionic polyoxyethylated fixed oils, such as Antarox (GAF Corp.), Emulphor (GAF Corp.), and Cremophor (BASF); and non-ionic polyoxyethylene sorbitan fatty acid ester derivatives, such as Tween 85, Tween 80, and Tween 40 (Atlas). The preferred surface-active agent is Antarox.

Suitable solvents include ethanol, propylene glycol, liquid low molecular weight polyethylene glycol and the like, with ethanol as the preferred solvent.

The liquified propellant employed is one which is a gas at room temperature (65°F.-80°F.) (18°C.-27°C.) and atmospheric pressure (760 mm. of mercury), i.e., it shall have a boiling point below 80°F. (27°C.) at atmospheric pressure. For use in compositions intended to produce aerosols for medicinal use, the liquified propellant should be non-toxic. Among the suitable liquified propellants which may be employed are the lower alkanes containing up to five carbon atoms, such as butane and pentane, or a lower alkyl chloride, such as methyl, ethyl or propyl chlorides. The most suitable liquified propellants are the fluorinated and fluorochlorinated lower alkanes such as are sold under the trademark "Freon." Mixtures of the above mentioned propellants may suitably be employed.

It is contemplated that the fluorinated or fluorochlorinated lower alkane shall contain not more than two carbon atoms and at least one fluorine atom. The preferred halogenated lower alkane compounds may be represented generally by the formula $C_mH_nCl_yF_z$, wherein m is an integer less than 3, n is an integer or zero, y

is an integer or zero, and z is an integer such that n+y+z=2m+2. Examples of these propellants are dichlorodifluoromethane ("Freon 12"), dichlorotetrafluoroethane ("Freon 114") $CClF_2CClF_2$, trichloromonofluoromethane ("Freon 11"), dichloromonofluoromethane ("Freon 21"), monochlorodifluoromethane ("Freon 22"), trichlorotrifluoroethane ("Freon 113"), and monochlorotrifluoromethane ("Freon 13"). Propellants with improved vapor pressure characteristics may be obtained by using certain mixtures of these compounds, e.g., "Freon 11" with "Freon 12," or "Freon 12" with "Freon 114." For example, dichlorodifluoromethane, which has a vapor pressure of about 70 pounds per square inch gauge ($5.84X10^5$ Pa) and 1,2-dichloro-1,1,2,2-tetrafluoroethane ("Freon 114"), with a vapor pressure of about 13 pounds per square inch guage ($1.91X10^5$ Pa) at 70°F. (21°C), may be mixed in various proportions to form a propellant having an intermediate vapor pressure, which is well suited for use in relatively low pressure containers.

It is most desirable that the vapor pressure of the propellant employed shall itself be between about 25 and 65 pounds per square inch gauge ($2.74X10^5$ - $5.49X10^5$ Pa) at 70°F. (21°C.), and preferably between about 30 and 40 pounds per square inch gauge ($3.08X10^5$ - $3.77X10^5$ Pa) at that temperature. A one-component propellant defined for use in the composition was found to give a composition with gauge pressures in the range of 55 to 65 pounds per square inch ($4.68X10^5$ - $5.49X10^5$ Pa) at 70°F. (21°C.), which are usable safely with metal containers. The two-component propellants, such as

equal weight mixtures of "Freon 12" and "Freon 11", were found to give gauge pressures in the range of 20 to 40 pounds per square inch ($2.39X10^5$ - $3.72X10^5$ Pa) at 70°F. (21°C.), which are usable safely with specially reinforced glass containers.

In preparing the stable solutions of this invention, minor amounts of conventional and commercially available pharmaceutical excipients (i.e., acceptable pharmaceutical grade preservatives, flavors, colors, and scents) can be employed, provided each is compatible with the solution. Exemplary of such excipients are optional preservatives selected from parabens (eg. propylparaben), benzyl alcohol, phenol and the like; flavors selected from menthol, peppermint oil, spearmint, and the like; colors selected from carmel, rose, and the like; and scenting agents selected from rose, lavender oil, and the like.

A preferred group of intranasal formulations are those containing the compounds of formula (I) wherein $R_2$ is phenyl, and which have a particle size from about 5 to about 20 microns.

Furthermore, the preferred compounds are substituted oxime derivatives of the benzimidazole, such as 1-isopropylsulfonyl-2-amino-5(6)-(α-hydroxyiminobenzyl)benzimidazole; 1-isopropylsulfonyl-2-amino-5(6)-(α-ethoxyiminobenzyl)benzimidazole; 1-isopropylsulfonyl-2-amino-5(6)-(α-acetoxyiminobenzyl)benzimidazole; and the like. The more preferred compounds are the syn- and anti-isomers of 1-isopropylsulfonyl-2-amino-6-(α-hydroxyiminobenzyl)benzimidazole; with the anti isomer most preferred.

A group of preferred formulations comprise, by weight:

a) from about 0.5 to about 1.0% of a compound of formula (I)

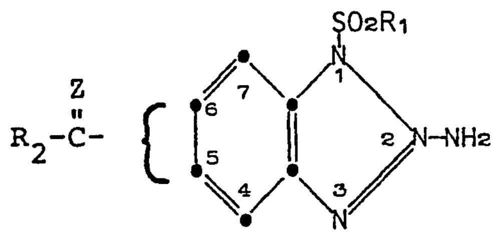

wherein $R_1$ is isopropyl, $R_2$ is phenyl and Z is hydroxyimino, $C_1$-$C_4$ acyloxyimino or $C_1$-$C_7$ alkylidene;

b) from about 20.0 to about 45.0% of the surface-active agent;

c) from about 5.0 to about 15.0% of the solvent; and

d) from about 40.0 to about 60.0% of the propellant.

Another group of preferred formulations comprise, by weight:

a) about 1.0% of 1-isopropylsufonyl-2-amino-6-(α-hydroxyiminobenzyl)benzimidazole;

b) about 39.0% of a polyoxyethylated fixed oil surface active agent;

c) about 25.0% of tricholoromonofluromethane and about 35.0% of dichlorodifluoromehtane.

A most preferred formulation comprises, by weight:

a) about 1.0% of 1-isopropylsulfonyl-2-amino-6-(α-hydroxyiminobenzyl)benzimidazole;

b) about 38.5% of a polyoxyethylated fixed

oil, surface-active agent;

c) about 10.0% of ethanol;

d) about 25.0% of trichloromonofluoromethane and about 25.0% of dichlorodifluoromethane; and

e) about 0.5% of menthol.

The formulations are generally prepared in the following manner: the active compound of formula (I) is dissolved in the surface-active agent, while any desirable excipients are dissolved in the solvent. The excipient-solvent solution and the compound-agent solution are then mixed together. Finally, the propellant is added.

The formulations of this invention are described in the following examples. The examples are illustrative of the formulations, but are not to be construed as limiting the invention. In the examples and throughout the specification, the quantities of material are expressed in terms of percentages by weight of the total composition.

## Example 1

| Ingredients | Percent |
|---|---|
| 1-isopropylsulfonyl-2-amino-6-(anti-α-hydroxyiminobenzyl)-benzimidazole | 1.0 |
| Antarox | 38.5 |
| Ethanol | 10.0 |
| Freon 11 | 25.0 |
| Freon 12 | 25.0 |
| Menthol | 0.5 |

Procedure:

An appropriate container is fitted with a mechanical stirrer and a cooling bath. Antarox is placed in the container and heated to about 40-60°C. The oxime is then added to the Antarox with mixing. Once the oxime has dissolved, the solution is cooled to room temperature using an ice-water bath. In a separate container, menthol is dissolved in ethanol and then the menthol-ethanol mixture is added with mixing to the oxime-Antarox solution.

An appropriate amount of solution is then placed in an aerosol container and chilled to about 0°C. The Freon propellant mixture is then added and the aerosol container is sealed with a valve. The container is brought to room temperature.

The following compositions are made by the same procedure as Example 1.

Example 2

| Ingredients | Percent |
|---|---|
| 1-isopropylsulfonyl-2-amino-6-(anti-α-hydroxyiminobenzyl)-benzimidazole | 0.5 |
| Antarox | 41.0 |
| Ethanol | 8.0 |
| Freon 11 | 25.0 |
| Freon 12 | 25.0 |
| Menthol | 0.5 |

## Example 3

| Ingredients | Percent |
|---|---|
| 1-isopropylsulfonyl-2-amino-6-(anti-α-hydroxyiminobenzyl)-benzimidazole | 0.25 |
| Antarox | 41.25 |
| Ethanol | 8.0 |
| Freon 11 | 25.0 |
| Freon 12 | 25.0 |
| Menthol | 0.50 |

## Example 4

| Ingredients | Percent |
|---|---|
| 1-isopropylsulfonyl-2-amino-6-(anti-α-hydroxyiminobenzyl)-benzimidazole | 0.1 |
| Antarox | 41.4 |
| Ethanol | 8.0 |
| Freon 11 | 25.0 |
| Freon 12 | 25.0 |
| Menthol | 0.5 |

## Example 5

| Ingredients | Percent |
|---|---|
| 1-isopropylsulfonyl-2-amino-6-(anti-α-hydroxyiminobenzyl)-benzimidazole | 1.0 |
| Antarox | 40.5 |
| Ethanol | 8.0 |
| Freon 11 | 25.0 |
| Freon 12 | 25.0 |
| Menthol | 0.5 |

Example 6

| Ingredients | Percent |
|---|---|
| 1-isopropylsulfonyl-2-amino-6-(anti-α-hydroxyiminobenzyl)-benzimidazole | 1.0 |
| Antarox | 33.5 |
| Ethanol | 15.0 |
| Freon 11 | 25.0 |
| Freon 12 | 25.0 |
| Menthol | 0.5 |

Example 7

| Ingredients | Percent |
|---|---|
| 1-(thienyl-2-ylsulfonyl)-2-amino-6-(α-acetoxyimino-benzyl)benzimidazole | 0.1 |
| Antarox | 41.4 |
| Ethanol | 8.0 |
| Freon 11 | 25.0 |
| Freon 12 | 25.0 |
| Menthol | 0.5 |

The following examples were prepared substantially according to the procedure of Example 1.

Example 8

| Ingredients | Percent |
|---|---|
| 1-Cyclohexylsulfonyl-2-amino-6-(α-ethoxyiminobenzyl)-benzimidazole | 1.0 |
| Tween 80 | 38.5 |
| Propylene glycol | 10.0 |
| Freon 11 | 25.0 |
| Freon 12 | 25.0 |
| Peppermint oil | 0.5 |

Example 9

| Ingredients | Percent |
|---|---|
| 1-isopropylsulfonyl-2-amino-6-(anti-α-hydroxyiminobenzyl)-benzimidazole | 1.0 |
| Emulphor | 38.5 |
| Ethanol | 10.0 |
| Freon 11 | 25.0 |
| Freon 12 | 25.0 |
| Menthol | 0.5 |

Example 10

| Ingredients | Percent |
|---|---|
| 1-isopropylsulfonyl-2-amino-6-(α-hydroxyiminobenzyl)-benzimidazole | 1.0 |
| Emulphor | 40.5 |
| Ethanol | 8.0 |
| Freon 11 | 25.0 |
| Freon 12 | 25.0 |
| Menthol | 0.5 |

Example 11

| Ingredients | Percent |
|---|---|
| 1-isopropylsulfonyl-2-amino-6-(α-hydroxyiminobenzyl)-benzimidazole | 0.5 |
| Antarox | 41.0 |
| Ethanol | 8.0 |
| Freon 11 | 25.0 |
| Freon 12 | 25.0 |
| Menthol | 0.5 |

Example 12

| Ingredients | Percent |
|---|---|
| 1-isopropylsulfonyl-2-amino -5-(α-acetoxyiminobenzyl)-benzimidazole | 1.0 |
| Emulphor | 40.5 |
| Propylene glycol | 8.0 |
| Freon 11 | 25.0 |
| Freon 12 | 25.0 |
| Menthol | 0.5 |

Example 13-16 were prepared substantially according to the following procedure:

An appropriate container is fitted with a mechanical stirrer and a cooling bath. The non-ionic surface-active agent is placed in the container and heated to about 40-60°C. The active compound of formula I is then added to the non-ionic suface-active agent with mixing. Once the active compound of formula I has dissolved, the solution is cooled to room temperature using an ice-water bath.

An appropriate amount of solution is then placed in an aerosol container and chilled to about -30°C. The Freon propellant mixture is then added and the aerosol container is sealed with a valve. The container is brought to room temperature.

Example 13

| Ingredients | Percent |
|---|---|
| 1-isopropylsulfonyl-2-amino -6-(anti-α-hydroxyiminobenzyl)-benzimidazole | 1.0 |
| Emulphor | 44.0 |
| Freon 11 | 15.0 |
| Freon 12 | 40.0 |

Example 14

| Ingredients | Percent |
|---|---|
| 1-isopropylsulfonyl-2-amino -6-(α-hydroxyiminobenzyl)-benzimidazole | 1.0 |
| Emulphor | 44.0 |
| Freon 11 | 20.0 |
| Freon 12 | 35.0 |

Example 15

| Ingredients | Percent |
|---|---|
| 1-isopropylsulfonyl-2-amino -6-(α-hydroxyiminobenzyl)-benzimidazole | 1.0 |
| Antarox | 49.0 |
| Freon 11 | 10.0 |
| Freon 12 | 40.0 |

Example 16

| Ingredients | Percent |
|---|---|
| 1-isopropylsulfonyl-2-amino -6-(anti-α-acetoxyiminobenzyl) benzimidazole | 1.0 |
| Emulphor | 49.0 |
| Freon 11 | 15.0 |
| Freon 12 | 35.0 |

## CLAIMS

1. An intranasal formulation which comprises, by weight:

a) from about .01 to about 1.0% of a compound having the formula (I)

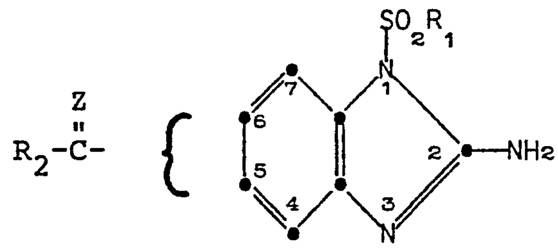 (I)

wherein $R_1$ is $C_1$-$C_5$ alkyl, $C_3$-$C_7$ cycloalkyl, or thienyl;
$R_2$ is hydrogen, $C_1$-$C_7$ alkyl, $C_3$-$C_7$ cycloalkyl, or phenyl;
Z is oxo, hydroxyimino, $C_1$-$C_4$ acyloxyimino, or $C_1$-$C_7$ alkylidene; and

$R_2-\overset{\overset{Z}{\|}}{C}-$ is at the 5 or 6 position;

such compound of formula (I) having a particle size from about 5 to about 200 microns;

b) from about 15.0 to about 50.0% of a pharmaceutically-acceptable, non-ionic, surface-active agent with a HLB number from about 12 to about 16;

c) from 0.0 to about 25.0% of a pharmaceutically-acceptable, solvent selected from ethanol, propylene glycol, and liquid polyethylene glycol; and

d) from about 40.0 to about 80.0% of a propellant.

2. The formulation of Claim 1 wherein the formulation comprises, by weight:

a) from about 0.5 to about 1.0% of the compound of formula (I);

b) from about 5.0 to about 15.0% of the surface-active agent;

c) from about 5.0 to about 15.0% of the solvent; and

d) from about 40.0 to about 60.0% of the propellant.

3. The formulation of Claim 1 wherein the formulation comprises, by weight:

a) from about 0.5 to about 1.0% of the compound of formula (I);

b) from about 5.0 to about 15.0% of the surface-active agent; and

c) from about 40.0 to about 60.0% of the propellant.

4. The formulation of Claim 1, 2, or 3 wherein the particle size of the compound is from 5 to about 20 microns.

5. The formulation of Claim 1, 2 or 3 wherein the surface-active agent is a polyoxyethylene sorbitan fatty acid ester derivative.

6. The formulation of Claim 1, 2 or 3 wherein the surface-active agent is a polyoxyethylated fixed oil.

7. The formulation of Claim 1, 2 or 3 wherein the propellant is a fluorochlorinated lower alkane.

8. The formulation of Claim 7 wherein the propellant is a mixture of trichloromonofluoromethane and dichlorodifluoromethane.

9. The formulation of Claim 1 or 2 wherein the solvent is ethanol.

10. The formulation of Claim 1, 2 or 3 wherein the formulation also contains a pharmaceutically-acceptable excipient.

11. The formulation of Claim 10 wherein the excipient is menthol.

12. The formulation of Claim 1, 2 or 3 which contains the compound of formula (I)

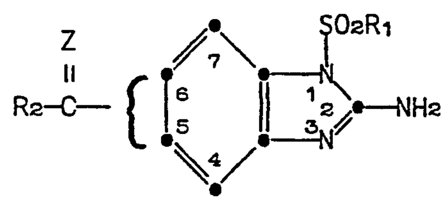

wherein $R_1$ is $C_1$-$C_5$ alkyl or thienyl; $R_2$ is phenyl; Z is hydroxyimino, $C_1$-$C_4$ acyloxyimino, or $C_1$-$C_7$ alkylidene; and

$R_2-\overset{Z}{\overset{\|}{C}}-$ is at the 6 position.

13. The formulation of Claim 12 wherein the compound is 1-isopropylsulfonyl-2-amino-6-(α-hydroxyiminobenzyl)benzimidazole.

14. The formulation of Claim 13 wherein the 1-isopropylsulfonyl-2-amino-6-(α-hydroxyiminobenzyl) benzimidazole is the anti isomer.

15. The formulation of Claim 13 wherein the isopropylsulfonyl-2-amino-6-(α-hydroxyiminobenzyl) benzimidazole is the syn isomer.